# EUROPEAN PATENT APPLICATION

(11) **EP 0 724 866 A1**
(43) Date of publication of application: **07.08.1996**
(21) Application number: 96300692.9
(22) Date of filing: 31.01.1996
(51) Int. Cl.: A61B 17/36, A61N 5/06

(54) **Laser facial rejuvenation**

(30) Priority: 03.02.1995 US 382918
(71) Applicant: LASER INDUSTRIES LIMITED, Tel Aviv 61131 (IL)
(72) Inventor: Slatkine Michael, 46427 Herziiah (IL); Mead Douglass, Allendale, New Jersey 07401 (US)
(74) Representative: Beresford, Keith Denis Lewis

(57) **Abstract**

A method and apparatus for performing facial rejuvenation are provided in which ablation of an area of skin is accomplished to the papillary dermis, providing effective permanent smoothness.

## Description

The present invention achieves rejuvenation of skin with minimal thermal damage and carbonization to the papillary dermis. Indications include the smoothing or rejuvenation of perioral, lips and periorbital wrinkles, among others.

Current treatments of the skin surface, whether for cosmetic or clinical applications, have not proven satisfactory. The most common current modalities of skin rejuvenation, namely chemical peeling and mechanical dermabrasion, suffer from lack of depth control and predictability. In addition, dermabrasion may result in bleeding which sends blood particles air-borne. Chemical peeling has the additional drawbacks of possible continued acid penetration after the chemicals are washed away and hypopigmentation.

An object of the present invention is to provide a method, and also apparatus, of applying a laser beam to a working surface such as to produce a substantially homogenous distribution of the laser energy over the surface of skin to be smoothed, particularly for facial rejuvenation. There is provided a method of applying a laser beam to a working surface, comprising: displacing the laser beam to trace a plurality of circular scans over the surface to be smoothed; and continuously varying the diameters of the circular scans to produce a substantially homogenous distribution of the laser energy over the surface to be smoothed.

According to further features in the preferred embodiments of the invention described below, the laser beam is displaced to trace the circular scans by deflecting the laser beam along two orthogonal axes by first and second deflector devices having axes perpendicular to each other, the deflector devices being oscillated by first and second motors operated at a phase difference of 90°. In the described preferred embodiment, the laser beam is of circular cross-section, and the minimum diameter of the circular scans is approximately equal to the diameter of the laser beam. Preferably, the diameter is at least 3.5 mm. The diameters of the circular scans are varied so that they partially overlap the same amount.

As will be described more particularly below, the diameters may be continuously varied to produce the substantially homogenous distribution of the laser energy by varying the frequency of oscillation, maximum value of voltage, or both frequency of oscillation and maximum value of voltage, of the deflector devices.

As will be shown more particularly below, the described method and apparatus enable homogenous scanning of the entire surface to be smoothed such as to make the method and apparatus particularly useful for the surgical smoothing of tissue by ablation while causing minimal thermal damage to the surrounding tissue, holes or cracks in the surrounding tissue, or char residue over the ablated area.

A method of smoothing or rejuvenating a predetermined area of human skin is provided comprising ablating the epidermal layer of a predetermined area of human skin with a laser beam and further ablating a portion of the dermal layer originally underlying said ablated epidermal layer to the papillary dermis. The method further comprises cleaning the area ablated to provide a clean ablated area and protecting the clean ablated area.

The method of smoothing a predetermined area of human skin involves, moreover, irradiating a portion of a predetermined area of human skin with a laser beam for a predetermined scan time to the papillary dermis, irradiating a next portion of said predetermined area of human skin for said predetermined scan time to the papillary dermis; and repeating the second and third steps such that the irradiating causes ablation of the skin uniformly.

An objective of the present invention is to provide a method of facial rejuvenation which achieves depth control of ablation to the papillary dermis with minimal thermal damage.

A further objective of the present invention is to provide a method of smoothing raised areas of skin which achieves depth control of ablation to the papillary dermis with minimal thermal damage.

A number of embodiments of the invention will now be described, by way of example only, with reference to the accompanying drawings in which:
Fig. 1 illustrates one form of apparatus constructed in accordance with an embodiment of the present invention;
Figs. 2-8 are diagrams helpful in explaining the operation of the apparatus of Fig. 1;
Fig. 9 depicts a fiber through which a laser beam may travel.

A laser beam is employed to rejuvenate or smooth the skin.

The embodiment of the present invention utilizes a laser preferably in conjunction with a flash scanner system. Flash scanner systems are described in U.S. Patent No. 5,411,502 entitled "A System for Causing Ablation of Irradiated Material of Living Tissue While Not Causing Damage Below a Predetermined Depth", and in the U.S. Patent Application No. 08/385,386 entitled "Method and Apparatus for Applying Laser Beam to a Working Surface, Particularly for Ablating Tissue". The flash scanner contains reflectors such as mirrors or prisms to reflect laser beams of light. The movements of the flash scanner are generally microprocessor controlled to provide the desired pattern of irradiation. The carbon dioxide laser is preferable for the uniform ablation of irradiated material. The laser beam of light may be emitted from articulated arm or, as provided herein, an optical waveguide. A focused or slightly defocused beam may be used.

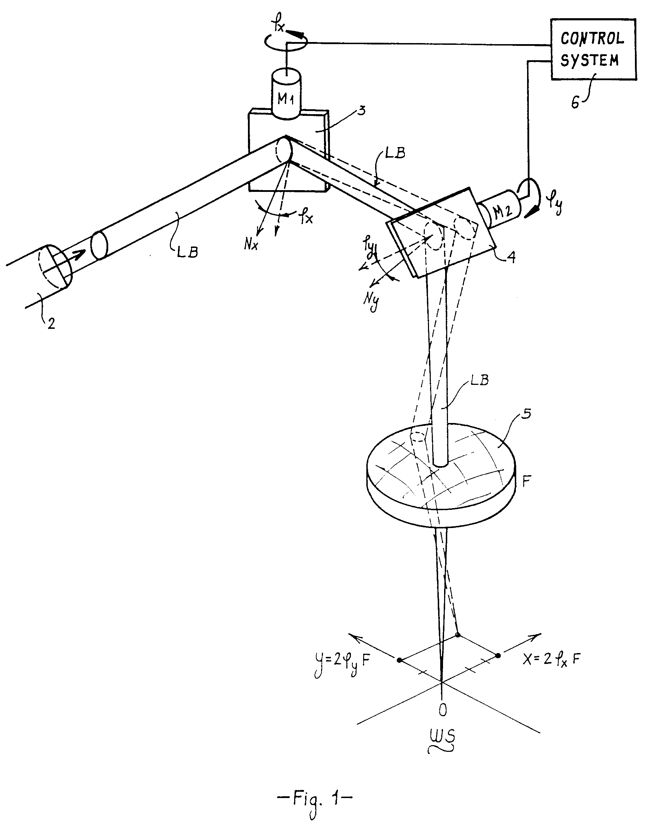

The apparatus illustrated in Fig. 1 includes a laser 2 outputting a laser beam LB which is directed to a working surface WS, such as tissue to be surgically smoothed by ablation. The laser beam LB from laser 2 is first deflected by a mirror deflector device 3, then by a mirror deflector device 4, which directs the beam via a focusing lens 5 to the working surface WS. Mirror 3 is oscillated along one axis, e.g., the X-axis, by a first motor M₁; and mirror 4 is oscillated along the other orthogonal axis, e.g., the Y-axis, by a second motor M₂. The two mirrors 3, 4, are located such that their axes (their normals Nx, Ny) are perpendicular to each other. Both motors are controlled by a control system, generally designated 6, in a manner to produce a homogenous scanning of the laser beam LB over the working surface WS, namely the tissue to be smoothed.

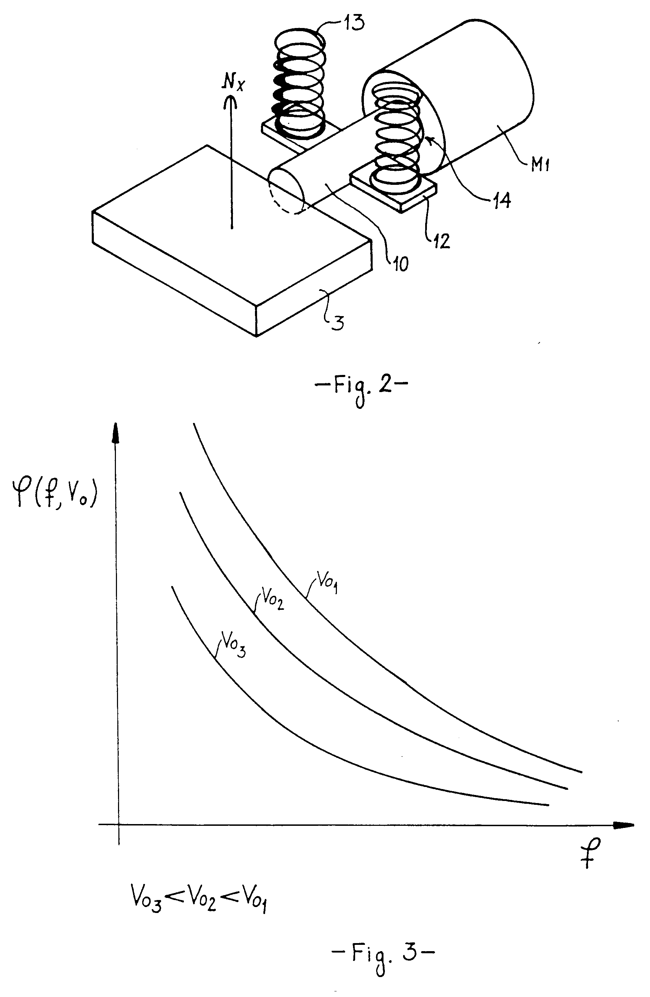

The following description, with reference particularly to Figs. 2-8, will explain how the motors M₁, M₂ are controlled to produce a homogenous scanning of the tissue to be smoothed.

Fig. 2 illustrates one of the oscillating mirrors, e.g., mirror 3, and its motor drive, e.g., motor M₁. Motor M₁ is connected via its rotary shaft 10 to mirror 3. The mirror's normal vector Nx is perpendicular to axis 10. Two arms 11, 12 are connected to axis 10 and press against two springs 13, 14, which are connected to the motor's housing. The two springs 13, 14 produce opposite torques on the motor's shaft 10, so that the shaft is at angular equilibrium.

When the motor M₁ is supplied with an electrical voltage V, it rotates the mirror 3 against the springs 13 14 until a new equilibrium is reached at a new angle φ in relation to the previous equilibrium point (φ = 0). A minus voltage (-V) will cause the motor and the mirror to rotate in the opposite direction at an angle of - φ. When the rate of voltage change is slow compared to the resonance frequency of the system, the angular displacement is linear to V; that is:$\text{φ = αV}$ where α is a proportional factor which is determined by the springs constants and the motor power.

When feeding the motor with alternating voltage, as described in the following equation:${\text{V(t) = V}}_{\text{o}} \text{Sin(2πft)}$ Where Vₒ is the maximum value of voltage, f the frequency, and t is the time; the motor, together with the mirror, oscillates clockwise and counterclockwise according to the equation:${\text{φ (t) = φ(V}}_{\text{o}} \text{, f)Sin(2πft+Φ)}$ where φ(Vₒ ,f) is the maximum displacement angle, which depends on Vₒ and the frequency f: and Φ is the phase between the mechanical oscillations and the electrical voltage.

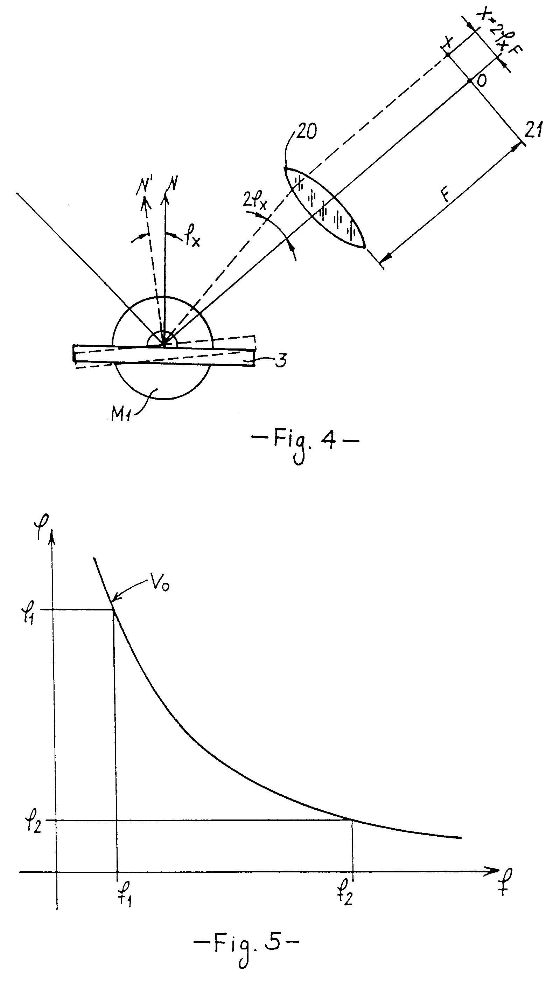

When using frequencies f, which are greater than the resonance frequency of the system, the maximal displacement angle φ(Vₒ ,f) can be described as a function off and Vₒ (see Fig. 3).

Each of the functions φ(f) in Fig. 3 is ascribed to a different Vₒ. An increase in Vₒ will cause an increase in displacement amplitude. The variation of the displacement amplitude may be controlled by one of the following methods:
1. Keep Vₒ constant and vary f;
2. Keep f constant and vary Vₒ; or
3. Vary both Vₒ and f.

Fig. 4 illustrates the deflection of a light beam by the scanner. When a light beam strikes mirror 3 at an angle of 45°, the beam is reflected and returned at an angle of 45°. If a lens 20 with a focal length of F is placed in the beam path, the beam will focus at a point O on the focal plane 21. This point O is referred to as the origin of the axis. When the mirror rotates at an angle of φₓ, the returned beam is reflected at an angle of 2φₓ and focused on the focal plane at point X, which is given by:${\text{X=2φ}}_{\text{x}} \text{F}$

When the two scanners are placed so that the two mirror normals Nx, Ny are perpendicular as shown in Fig. 1, voltage supplied to the two motors (M₁, M₂) will cause movement at the focal plane in both x and y directions as follows:$\begin{matrix}\begin{matrix}{\text{X = 2φ}}_{\text{x}} \text{F} \\ {\text{Y = 2φ}}_{\text{y}} \text{F}\end{matrix}\end{matrix}$ If the motors are provided with an alternating voltage at a frequency f and an amplitude Vₒ, but with a phase difference of 90°, then:$\begin{matrix}\begin{matrix}{\text{V}}_{\text{x}} {\text{= V}}_{\text{o}} \text{Sin(2πft)} \\ {\text{V}}_{\text{y}} {\text{= V}}_{\text{o}} \text{Cos(2πft)}\end{matrix}\end{matrix}$ After substituting in Equations (3) and (5), the coordinates will be as follows:$\begin{matrix}\begin{matrix}{\text{x(t) = 2F (V}}_{\text{o}} \text{,f)sin(2πft)} \\ {\text{y(t) = 2F (V}}_{\text{o}} \text{,f)cos(2πft)}\end{matrix}\end{matrix}$ These equations form a circle with a radius of:${\text{r= √x}}^{\text{2}} {\text{+y}}^{\text{2}} {\text{= √8Fφ(V}}_{\text{o}} \text{,f)}$ The velocity on the circumference will be 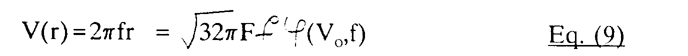 The velocity is constant for the circumference.

The radius of the scanning can be controlled by changing φ(Vₒ,f). This can be done by: (1) varying f when Vₒ is constant, (2) by varying Vₒ when f is constant, or (3) by varying both.

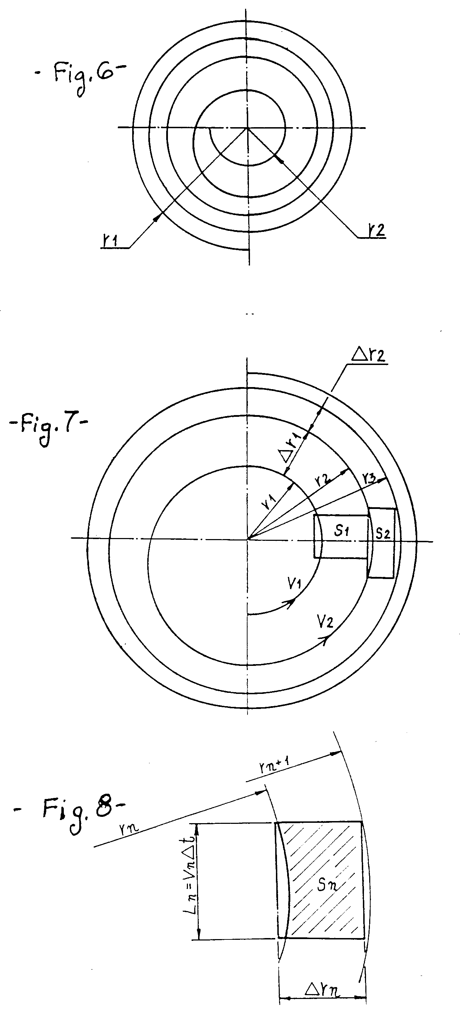

To scan a full area (not just the perimeter), a constant voltage amplitude Vₒ with a curve described in Fig. 6 may be supplied to the two motors. Operating the motors at a frequency f₁ will cause rotational amplitude φ₁ and circular scanning according to Equation (8) at a radius of ${\text{r}}_{\text{1}} {\text{= √8 Fφ}}_{\text{1}} {\text{(V}}_{\text{o}} {\text{,f}}_{\text{1}} \text{)}$, Gradually increasing the frequency to f₂ will cause the rotational amplitude to gradually decrease to the value φ₂(Vₒ,f₂) and the radius of the circular scanning to ${\text{r}}_{\text{2}} {\text{= √8 Fφ}}_{\text{2}} {\text{(V}}_{\text{o}} {\text{,f}}_{\text{2}} \text{)}$ where r₂<r₁.

On the focal plane, circles are formed with gradually decreasing radii from r₁ to r₂, thereby covering an area of a ring with an outer radius of r₁ and an inner radius of r₂ (see Fig. 6). Gradually decreasing the frequency to f₁ will produce an area scanning with circles of gradually increasing radii to a radius of r₁. Alternately varying frequencies between f₁ and f₂ will cause area scanning by means of continuously increasing and decreasing circles. The boundaries of the scanning (outer and inner radii) are determined by the extreme values of frequencies, f₁ and f₂. The control system 6 (Fig. 1) can be easily computerized such that the user selects the diameter of scanning, and the system determines the required frequencies according to the above equations.

Since the difference between two following scanning radii decreases, the energy per unit area increases. The difference between two consecutive scanning radii can be controlled by the rate of change of f. Since the frequency f is increased or decreased for each circular scanning, the difference between two consecutive circles will increase or decrease. Controlling the rate that frequency f is changed throughout each scanning, enables the control of radial energy distribution.

The scanned area is described in Fig. 8. S₁ and S₂ represent block areas. The area width always is the difference of radii between two consecutive scannings, and their height is the displacement L that the beam has traversed at unit time Δt. The displacement will be:$\text{L=V(r)Δt}$ wherein:
- P =: laser power
- E =: energy radiated onto block area S
- rₙ =: radius of scanning corresponding to Sₙ
- Δrₙ =: the difference between rₙ and the consecutive radius ${\text{r}}_{\text{n+1}} {\text{(Δr}}_{\text{n}} {\text{=r}}_{\text{n+1}} {\text{- r}}_{\text{n}} \text{)}$
- V(r) =: linear velocity of the scanning beam
- Δt =: time unit determined for all areas
- Lₙ =: height of the block area corresponding to rₙ
- σₙ =: energy density per unit time in a block area corresponding to radius rₙ

The energy density may be calculated as follows:${\text{σ}}_{\text{n}} \text{=} \frac{\text{E}}{{\text{S}}_{\text{n}}} \text{=} \frac{\text{E}}{{\text{L}}_{\text{n}} {\text{·Δr}}_{\text{n}}} \text{=} \frac{\text{PΔt}}{{\text{V(r}}_{\text{n}} {\text{)·Δt·Δr}}_{\text{n}}} \text{= P} \frac{\text{1}}{{\text{V(r}}_{\text{n}} {\text{)Δr}}_{\text{n}}}$

Assuming that the laser power is constant throughout scanning, the power density per unit area is proportional to 1/V(rₙ) Δrₙ. To achieve constant energy density for the entire scanning area (homogenous ablation), V(rₙ) Δrₙ should be kept constant. From Equation 9:${\text{f}}_{\text{n}} {\text{= (V}}_{\text{o}} {\text{,f}}_{\text{n}} {\text{)·Δr}}_{\text{n}} \text{=CONSTANT}$

Following is one procedure for producing a constant energy density wherein the frequency of the two motors M₁, M₂ is controlled by the control system 6 (Fig. 1). The minimum diameter of the circular scans is equal to the diameter of the light beam LB, which is preferably at least 3.5 mm; and the diameters of the circular scans are varied such that they partially overlap the same amount:
1) The minimum radius r₂ is determined for the first scanning circle, according to the radius of the focused laser beam.
2) The first radii difference Δr₁, between the first and second scanning circles, is determined at a value of 3/4 of the radius of the focused laser beam, so that the scanning circles overlap.
3) A curve φ(Vₒ,f) is determined by selecting Vₒ. From this curve, frequency f₁ which corresponds to r₁, and frequency f₂ which corresponds to ${\text{r}}_{\text{2}} {\text{=r}}_{\text{1}} {\text{+Δr}}_{\text{1}}$, are chosen.
4) The product ${\text{C=φ(V}}_{\text{o}} {\text{,f) Δr}}_{\text{1}} {\text{f}}_{\text{1}}$ is calculated (Equation 12).
5) Δr₂ is calculated from Equation 12 ${\text{Δr}}_{\text{2}} {\text{=C/φ(V}}_{\text{o}} {\text{,f}}_{\text{2}} {\text{) f}}_{\text{2}}$.
6) The new radius is ${\text{r}}_{\text{3}} {\text{=r}}_{\text{2}} {\text{+Δr}}_{\text{2}}$, and f₃ is chosen from the curve.
7) Δr₃ is calculated ${\text{Δr}}_{\text{3}} {\text{=C/φ(V}}_{\text{o}} {\text{,f}}_{\text{3}} {\text{)f}}_{\text{3}}$, and so on until the outer radius is reached.

The sequence of frequencies (or matching time periods) in a phase difference of 90° are supplied by the control system 6 in Fig. 1 to the two motors M₁, M₂, thereby producing area scanning with uniform energy density for the entire area scanned.

Power density may also be controlled by varying Vₒ and keeping f constant, or by varying both. Any desired radial energy distribution may be programmed except for constant radial energy distribution.

Alternatively, the scanning system may trace lissajous figures as disclosed in U.S. Patent No. 5,411,502. In such a system the mirrors are located with respect to the laser beam and also to each other to cyclically scan the laser beam along two orthogonal axes, and to cause the beam to trace a Lissajous pattern over the tissue to be smoothed.

Where the lens is placed perpendicular to the axis of the laser beam, a time dependent ray pattern will be produced at the focal plane of the lens (of focal length f), given by the following equations:$\begin{matrix}\begin{matrix}{\text{}}_{\text{x2}} {\text{(t)=fa}}_{\text{x2(t)}} {\text{=f(θ/√2)cos(Ω}}_{\text{1}} {\text{*t)+fθ cos (Ω}}_{\text{2}} \text{*t)} \\ {\text{}}_{\text{y2}} {\text{(t)=fa}}_{\text{y2(t)}} {\text{=fθ sin (Ω}}_{\text{1}} {\text{*t)+f(θ/√2)sin (Ω}}_{\text{2}} \text{*t)}\end{matrix}\end{matrix}$

For example, the lens may be of f=125 mm; the mirror wedge angle may be ⊖=2.34 mRad; and the angular velocities may be Ω1=600 rad/sec and Ω2=630 rad/-sec. Let $\text{A=θ/√2=0.207}$; $\text{B=θf-0.293}$; and $\text{C=Ω2/Ω1=1.05}$. The ray exiting from the lens will scan at the focal plane an area whose limits are defined by a circle of radius 0.5 mm. Every 20 revolutions the ray completely scans the whole area and starts anew. The 20 revolution scan period is about 0.2 seconds and a lissajous pattern is achieved.

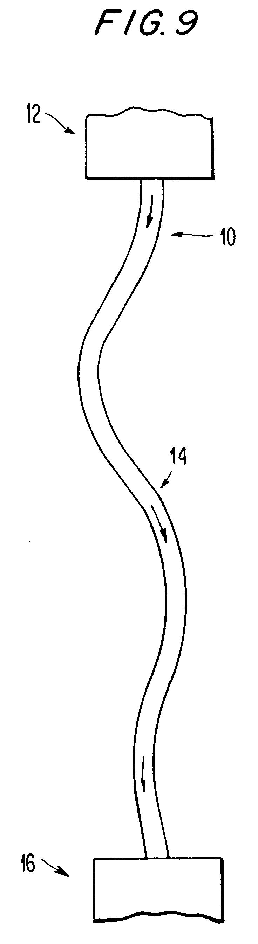

With respect to performing laser facial rejuvenation, the beam preferably travels through an optical waveguide before reaching the flash scanner. Fig. 9 depicts such an optical waveguide 10 through which a laser beam may travel. The laser beam is generated at laser source 12 and travels through the optical waveguide 14 in the direction of the arrows to the flash scanner 16 containing the reflector system. The optical waveguide, which is loosely referred to as a fiber, provides superior waveguide capability for the laser beam. It also participates in defocusing the laser beam. After passing through the flash scanner, the laser beam is emitted to irradiate the skin surface (not shown here).

The present invention permits all irradiated skin to be ablated with negligible thermal damage and char to the underlying skin. Moreover, any residual thermal damage is shallow and controlled.

The use of flash scanning in facial rejuvenation enables the smoothing of raised areas of the skin such as wrinkle shoulders around the mouth and eyes as well as scars and warts. The epidermal areas and underlying dermal layers are vaporized layer by layer. The treatment can be performed using a predetermined pattern of a spiral pattern or Lissajous figures. A spiral pattern is preferable for skin resurfacing as homogeneous vaporization is particularly desirable for cosmetic or aesthetic surgeries. As described herein, the flash scanner is preferably used in conjunction with scanning for a predetermined scan time.

The method of the present invention provides that the entire epidermal layer of skin of the affected area is ablated. Then ablation proceeds to the papillary dermal layer to a depth of about 70-200 microns, to above the collagen producing cells of the papillary dermis. At typical 7 watt laser operating power, for instance, depth is typically 70 microns with residual thermal damage to the subjacent dermis as low as 75-100 microns. The minimal thermal necrosis resulting to this portion of the dermal layer permits collagen production for smoothing out the skin so as to provide sufficient healing with substantially permanent results. Such favorable cosmetic or aesthetic treatments on otherwise healthy tissue is made possible by this technique.

Rapid movement of the beam over the tissue ensures a 1-2 millisecond short duration of exposure on individual sites within the area. This is shorter than the thermal relaxation time of tissue for laser beam penetration depth of 30 micron at 10.6µ wavelength. The desired scan time is preprogrammed. Irradiation proceeds beginning at another location with minimal overlapping. In this manner the depth of irradiation can be accurately predetermined. The spot size of the laser beam on the skin may be from somewhat less than 0.20 mm diameter to 0.6 mm diameter. The area treated may, for instance, be up to 6 or 9 mm in diameter, with power requirements increasing accordingly.

Advantageously, no bleeding results from the treatment. Following ablation, residual coagulated gray epidermal tissue is wiped off with sterile, saline soaked gauze to expose the dermal layer which is then protected and moisturized with dressings.

The method of the present invention is optimal for facial rejuvenation, for example, the smoothing of wrinkles. The method may also be used for skin rejuvenation as may be contemplated by those skilled in the art.

### Example

In the following manner the single layer vaporization depth was quantitatively estimated. A CO₂ laser was used in clinical cases which generated a focused beam somewhat less than 0.2 mm diameter on tissue. Using the laser at a power level of 7W for facial rejuvenation will generate an optical power density of above 100 W/mm² on tissue. This is considerably higher than the threshold for vaporization of tissue without residual carbon particles (the threshold for char-free tissue ablation is about 50 W/mm² ). The time required to homogeneously cover a round area was programmed to be 200 msec. During this time, the 7W operating laser delivers 1400mJ to the tissue. Since the typical energy required to completely ablate tissue is about 3000mJ for 1mm³ volume, keeping the facial skin resurfacing handpiece precisely on a single site for 0.2 sec will generate a clean charfree crater of less than 70 micrometer depth for 3mm diameter scanned area. Minimal residual thermal reaction resulted to the papillary an reticular dermis. No damage occurred to adnexal structures. Histologies of excised facial skins ablated as described also showed that the thermal subcrater necrosis depth was less than 150µm.

Over 50 skin exfoliations were performed to smooth out perioral, lip and periorbital wrinkles and scars. The laser power level was set to approximately 7 Watts, although the precise power level selected depended on the skin thickness in conjunction with skin darkness and hair color. In treating wrinkles, the "shoulders" were ablated along both sides of the wrinkles by spiral pattern with caution to avoid overlapping treatment spots and thus avoid ablation of the papillary dermis. The laser repeat mode was used with the laser set to 0.2 sec "on time" and 0.4 sec "off time." Following ablation, residual coagulated gray epidermal tissue was wiped off with sterile, saline soaked gauze to expose the dermal layer which was then protected and moisturized with dressings. Full healing was attained within three months. No permanent hyer- or hypo- pigmentation was observed, although the skin appeared characteristically "pink" for about six weeks.

The entire procedure may be done in an office setting under local anesthesia and lasts 20 minutes on average.

## Claims

1. A method of smoothing a predetermined area of human skin comprising:
scanning the epidermal layer of said predetermined area of human skin sequentially and continuously with a laser beam; and
scanning a portion of the dermal layer underlying said ablated epidermal layer to the papillary dermis such that collagen producing cells of the papillary dermis retain the capability of collagen production.

2. The method as claimed in Claim 1 further comprising:
cleaning the area scanned to provide a clean scanned area; and
protecting said clean scanned area.

3. A method of smoothing a predetermined area of human skin comprising:
scanning a portion of said predetermined area of human skin continuously with a laser beam for a predetermined scan time to the papillary dermis such that the collagen producing cells of the papillary dermis underlying said portion of a predetermined area of human skin retain the capability of collagen production; and
scanning a next portion of said predetermined area of human skin continuously with a laser beam for a predetermined scan time to the papillary dermis such that the collagen producing cells of the papillary dermis underlying said next portion of a predetermined area of human skin retain the capability of collagen production.

4. The method as claimed in Claim 1 in which said laser is a carbon dioxide laser.

5. The method as claimed in Claim 3 in which said laser is a carbon dioxide laser.

6. The method as claimed in Claim 1 further comprising passing a laser beam through an optical waveguide prior to scanning said epidermal layer with said laser beam.

7. The method as claimed in Claim 3 further comprising passing a laser beam through an optical waveguide prior to scanning said laser beam.

8. The method as claimed in Claim 1 in which said dermal layer is ablated to a depth of about 70-200 microns.

9. The method as claimed in Claim 3 in which the dermal layer of each portion of human skin scanned is ablated to a depth of about 70-200 microns.

10. An apparatus suitable for smoothing a predetermined area of human skin comprising:
a laser for providing a laser beam; and
a flashscanner capable of scanning said beam continuously over a predetermined area of human skin to the papillary dermis such that the collagen producing cells of the papillary dermis retain the capability of collagen production.

11. The apparatus as claimed in Claim 10 in which the dermal layer is ablated to a depth of between about 70-200 microns.

12. The apparatus as claimed in Claim 10 in which thermal damage occurs to the subjacent dermis to a depth of 75-100 microns.

13. The apparatus as claimed in Claim 10 in which said laser is a carbon dioxide laser.

14. The apparatus as claimed in Claim 13 in which said laser is powered at 7 Watts.

15. The apparatus as claimed in Claim 10 in which said flashscanner scans said beam in a spiral pattern.

16. The apparatus as claimed in Claim 10 further comprising an optical waveguide capable of guiding the laser beam to said predetermined area of human skin.

17. An apparatus suitable for smoothing a predetermined area of human skin comprising:
a laser for providing a laser beam; and
a flashscanner capable of scanning said beam continuously and sequentially over a portion of said predetermined area of human skin to the papillary dermis such that the collagen producing cells of the papillary dermis underlying said portion of a predetermined area of human skin retain the capability of collagen production, where said flashscanner is capable of said scanning of said beam over a plurality of portions of said predetermined area of human skin.
